# EUROPEAN PATENT SPECIFICATION

(11) **EP 2 990 484 B1**
(45) Date of publication and mention of the grant of the patent: **10.04.2019**
(21) Application number: 14788079.3
(22) Date of filing: 23.04.2014
(51) Int. Cl.: C12N 15/09, C07K 7/06, C07K 7/08, C07K 14/00, C12N 1/15, C12N 1/19, C12N 1/21, C12N 5/10, C12Q 1/02, C40B 30/04, C40B 40/10

(54) **PEPTIDE-PRESENTING PROTEIN AND PEPTIDE LIBRARY USING SAME**
PEPTID-PRÄSENTIERENDES PROTEIN UND PEPTIDBIBLIOTHEK, DIE DIESES VERWENDET
PROTÉINE PRÉSENTANT UN PEPTIDE ET BANQUE DE PEPTIDES L'UTILISANT

(30) Priority: 23.04.2013 JP 2013090524
(43) Date of publication of application: 02.03.2016
(73) Proprietor: Tokyo Institute of Technology, Tokyo 152-8550 (JP)
(72) Inventor: KADONOSONO, Tetsuya, Tokyo 152-8550 (JP); KONDOH, Shinae, Tokyo 152-8550 (JP)
(74) Representative: Gille Hrabal
(86) International application number: PCT/JP2014/061366
(87) International publication number: WO 2014/175305

(56) References cited:
- WO-A1-99/24617
- JP-A- 2003 235 577
- JP-A- 2007 151 479
- US-A1- 2012 034 619
- N. VELAPPAN ET AL: "A comprehensive analysis of filamentous phage display vectors for cytoplasmic proteins: an analysis with different fluorescent proteins", NUCLEIC ACIDS RESEARCH, vol. 38, no. 4, E22, 2 December 2009 (2009-12-02), pages 1-16, XP055327067, GB ISSN: 0305-1048, DOI: 10.1093/nar/gkp809
- TETSUYA KADONOSONO ET AL: "A Fluorescent Protein Scaffold for Presenting Structurally Constrained Peptides Provides an Effective Screening System to Identify High Affinity Target-Binding Peptides", PLOS ONE, vol. 9, no. 8, 1 August 2014 (2014-08-01), page e103397, XP055327040, DOI: 10.1371/journal.pone.0103397
- PEDELACQ, JEAN-DENIS ET AL.: 'Engineering and characterization of a superfolder green fluorescent protein' NATURE BIOTECHNOLOGY vol. 24, no. 1, January 2006, pages 79 - 88, XP002368364
- ETSURI YABE ET AL.: 'Apoptosis Shinko o Kenshutsu suru Shinki Keiko Tanpakushitsu no Kaihatsu' ANNUAL MEETING OF THE MOLECULAR BIOLOGY SOCIETY OF JAPAN PROGRAM.YOSHISHU (WEB vol. 34, December 2011, page 1P-0876, XP008183372

## Description

### TECHNICAL FIELD

The present invention relates to a peptide-presenting protein and a peptide library using the same. According to the present invention, peptide(s) highly specific to a target molecule can be screened.

### BACKGROUND ART

Functional peptide(s) having high specificity to a target molecule and binding to a target molecule is useful for a drug-discovery. Thus, in order to obtain the functional peptide(s), many peptide libraries according to various peptide-presenting techniques have been constructed and many high-throughput screening techniques have been developed. As for the methods for presenting the peptide library, display on a phage, E.coli, a yeast, or the like is used. In the phage display, for example, screened peptides are presented as a fusion polypeptide on a bacteriophage surface protein. The peptide-displayed phage particles are brought into contact with a microtiter plate in which the target molecule is immobilized, to thereby conduct an affinity selection. Phages expressing peptide having affinity to target molecule can be concentrated due to this (non-patent literature 1).

In the phage display, the peptide presented on the concentrated phages corresponds one-to-one to a gene cording the same, and therefore, the peptide of interest can be easily identified. Further, the gene can be easily amplified (proliferated), and therefore, the phage display is widely used as the method for screening peptides.

### CITATION LIST

### NON-PATENT LITERATURE

[NON-PATENT LITERATURE 1] Science (United States) 1990, Vol. 249, p. 386-390
[NON-PATENT LITERATURE 2] Nature Biotechnology (Great Britain) 2006, Vol. 24, p. 79-88

### SUMMARY OF INVENTION

### TECHNICAL PROBLEM

However, peptide(s) obtained by the conventional display method can be bound to the target molecule, but are often bound to other molecules. That is to say, peptide(s) having low specificity to the target molecule may be screened, and thus, the peptide(s) cannot be frequently made fit for practical use as a medicine.

Accordingly, the object of the present invention is to provide a method for screening peptide(s) highly specific to a target molecule.

### SOLUTION TO PROBLEM

The inventors have conducted intensive studies for the low specificity of the screened peptide(s), and as a result, think that the peptide(s) presented by the conventional display method are screened under the condition that a structure of the peptide is freely changed. Thus, the inventors think that the many peptide(s) obtained as a candidate peptide may also bind to molecules other than the target molecule.

Further, the inventors of the present invention have conducted intensive studies into a method for limiting a flexibility of structure of the presented peptide, and as a result, surprisingly found that peptide(s) capable of specifically binding to the target molecule can be screened by inserting a peptide into the region consisting of the 131st to 139th amino acids of the superfolder GFP(non-patent literature 2; hereinafter sometimes referred to as sfGFP) to limit the flexibility of peptide structure.

The present invention is based on the above findings.

Namely, the present invention relates to:
[1] a peptide-presenting protein wherein a peptide is presented in a presentation region consisting of the 131st to 139th amino acids in
   (1) the amino acid sequence of the SEQ ID NO:54 or
   (2), wherein the amino acid sequence of SEQ ID NO:54, wherein one to three amino acids are substituted in the first to 130^{th} amino acids sequence or the 140^{th} to 238^{th} amino acids sequence,
      wherein the peptide consisting of four to fifteen amino acids is inserted in the 131st to 139th amino acids sequence, or substituted for any of 1 to 9 amino acid(s) sequence in the 131st to 139th amino acids sequence; and a total length of an amino acid sequence corresponding to the 131st to 139th amino acids sequence is fifteen or less, and the peptide exhibits a fluorescence generation activity.
[2] a fusion protein wherein the peptide-presenting protein of item [1], and a surface protein are fused,
[3] a polynucleotide encoding the peptide-presenting protein of item [1], or the fusion protein of the item [2],
[4] a vector comprising the polynucleotide of the item [3],
[5] a host comprising the vector of the item [4],
[6] a library comprising the host of the item [5], wherein the presented peptides are a number of peptides consisting of random amino acid sequences,
[7] a method for constructing a library, comprising the steps of:
   (1) inserting a polynucleotide encoding a peptide consisting of 4 to 15 amino acids into a vector comprising a polynucleotide encoding a protein for presenting a peptide or a fusion protein wherein the protein for presenting a peptide is fused with a surface protein, to obtain an expression vector, wherein the peptide is inserted into the 131st to the 139th amino acids sequence of the presentation region, or substituted for any of 1 to 9 amino acids sequence in the 131st to 139th amino acids sequence of the presentation region; and a total length of amino acids sequence corresponding to the 131st to 139th amino acid sequence is fifteen or less, and
   (2) introducing the vector into a host,
      wherein the protein for presenting a peptide presents the peptide in a presentation region consisting of the 131st to 139th amino acids in the amino acid sequence of the SEQ ID NO.54, wherein the peptide consisting of four to fifteen amino acids is inserted in the 131st to 139th amino acids sequence, or substituted for any of 1 to 9 amino acids(s) sequence corresponding to the 131st to 139th amino acids sequence; and a total length of amino acids sequence corresponding to the 131st to 139th amino acids sequence is fifteen or less.
[8] a method for screening a peptide binding to a target molecule, comprising the steps of:
   (1) bringing into contact the peptide library according to [6] with the target molecule,
   (2) recovering hosts binding to the target molecule.
[9] the method for screening a peptide according to [8], further comprising (3) the step of proliferating the recovered hosts.
[10] The method for screening a peptide according to [9], wherein the contact step (1), the host recovering step (2), and the proliferating step (3) are repeated.

### ADVANTAGEOUS EFFECTS OF INVENTION

According to the peptide-presenting protein and the peptide library using the same of the present invention, the peptide(s) highly specific to a target molecule can be screened. Further, the scaffold protein, i.e. sfGFP, in which the peptide is presented, is a fluorescence protein, and thus, the number of molecules thereof can be easily estimated by fluorescence intensity. Therefore, it is useful for estimating a binding amount of peptide in the screening. The present invention can be applied to the widely used presenting techniques, such as the phage display, E.coli display, yeast display, in vitro virus, and ribosome display. That is, the present invention can be applied to the conventional, high-throughput screening techniques. Furthermore, molecularly-targeted drugs or drug delivery systems can be developed based on the sequence information and structural information of a peptide obtained by the present invention.

### BRIEF DESCRIPTION OF THE DRAWINGS

FIG. 1 is an illustration showing an amino acid sequence of full length sfGFP, and a 131st to 139th amino acids sequence (reversed characters).
FIG. 2 is a photograph showing that the peptides recognized by caspase 3, which are introduced in Examples 1 to 15 and Comparative Examples 1 to 5, are cleaved by caspase 3.
FIG. 3 is a graph showing fluorescence intensities of peptide-presenting proteins obtained in Examples 1 to 15 and Comparative Examples 1 to 5.
FIG. 4 is illustrations showing molecular dynamics simulations of the peptides (m1 (Example 1), m6 (Example 4), m19 (Example 13), and m7 (Comparative Example 3)) inserted in the peptide-presenting protein.
FIG. 5 is a table showing the peptide sequences capable of binding to a HER2 protein, which is frequently overexpressed in breast cancer cells (A), and photographs showing that the peptide-presenting proteins wherein the above peptides are introduced, bind to HER2.
FIG. 6 is an illustration showing amino acid sequences of the peptide-presenting proteins of the present invention, i.e. m12 (Example 6), m15 (Example 9), m16 (Example 10), m19 (Example 13), m21 (Example 15), and m22 (Example 17) (A), and molecular dynamics simulations thereof (B).

### [1] Peptide-presenting protein, polynucleotide, vector, and host

### <<Peptide-presenting protein>>

In the peptide-presenting protein of the present invention, a peptide is presented in a presentation region consisting of the 131st to 139th amino acids in the amino acid sequence of the SEQ ID NO: 54, and the peptide consisting of four to fifteen amino acids (preferably 4 to 14 amino acids, more preferably 4 to 13 amino acids, most preferably 4 to 12 amino acids) is inserted in the 131st to 139th amino acids sequence, or substituted for any of 1 to 9 amino acid(s) sequence in the 131st to 139th amino acids sequence; and a total length of amino acids sequence corresponding to the 131st to 139th amino acids sequence is fifteen or less (preferably 14 or less, more preferably 13 or less, most preferably 12 or less). Further, a lower limit of the length of amino acids sequence corresponding to the 131st to 139th amino acids sequence is preferably five or more. The peptide-presenting protein of the present invention may be one wherein one to three amino acids are substituted in the first to 130th amino acids sequence or the 140th to 238th amino acids sequence in the amino acid sequence of SEQ ID NO: 54, and which exhibits a fluorescence generation activity. The protein consisting of the amino acid sequence of the SEQ ID NO: 54 is the sfGFP described in Non-patent literature 2. However, GFP-based proteins can be used as the scaffold protein for inserting the peptide in the peptide-presenting protein of the present invention. That is to say, GFP or GFP-based variant protein derived from GFP can be used without limitation. As a concrete scaffold protein, there may be mentioned GFP, YFP, CFP, BFP, or RFP.

Preferably, in the peptide-presenting protein of the present invention, a peptide is presented in a presentation region consisting of the 131 st to 138th amino acids in the amino acid sequence of the SEQ ID NO:54, and the peptide consisting of four to fourteen amino acids (preferably 4 to 13 amino acids, more preferably 4 to 12amino acids) is inserted in the 131st to 138th amino acids sequence, or substituted for any of 1 to 8 amino acid(s) sequence in the 131st to 138th amino acids sequence; and a total length of amino acids sequence corresponding to the 131st to 138th amino acids sequence is fourteen or less (preferably 13 or less, more preferably 12 or less). Further, a lower limit of the length of amino acids sequence corresponding to the 131st to 138th amino acids sequence is preferably four or more. The peptide-presenting protein of the present invention may be one wherein one to three amino acids are substituted in the first to 130th amino acids sequence or the 139th to 238th amino acids sequence in the amino acid sequence of SEQ ID NO:54, and which exhibits a fluorescence generation activity.

More preferably, in the peptide-presenting protein of the present invention, a peptide is presented in a presentation region consisting of the 131st to 137th amino acids in the amino acid sequence of the SEQ ID NO: 54, and the peptide consisting of four to thirteen amino acids (preferably 4 to 12 amino acids) is inserted in the 131st to 137th amino acids sequence, or substituted for any of 1 to 7 amino acid(s) sequence in the 131st to 137th amino acids sequence; and a total length of amino acids sequence corresponding to the 131st to 137th amino acids sequence is thirteen or less (preferably 12 or less). Further, a lower limit of the length of an amino acids sequence corresponding to the 131st to 137th amino acids sequence is preferably four or more. The peptide-presenting protein of the present invention may be one wherein one to three amino acids are substituted in the first to 130^{th} amino acids sequence or the 139th to 238th amino acids sequence in the amino acid sequence of SEQ ID NO:54, and exhibiting a fluorescence generation activity.

In connection to this, the presented peptide having a shorter length is preferable, because an aggregation of the protein may be suppressed. Further, as to the presentation region, the region consisting of the 131st to 138th amino acids, or the region consisting of the 131st to 137th amino acids is preferable, because an aggregation of the protein may be suppressed, compared to the region consisting of the 131 st to 139th amino acids.

### <<Protein for presenting a peptide>>

The protein for presenting a peptide of the present invention can present a peptide in a presentation region consisting of the 131st to 139th amino acids in the amino acid sequence of the SEQ ID NO: 54, and the peptide consisting of four to fifteen amino acids (preferably 4 to 14 amino acids, more preferably 4 to 13 amino acids, most preferably 4 to 12 amino acids) is inserted in the 131st to 139th amino acids sequence, or substituted for any of 1 to 9 amino acid(s) sequence in the 131st to 139th amino acids sequence; and a total length of amino acids sequence corresponding to the 131st to 139th amino acids sequence is fifteen or less (preferably 14 or less, more preferably 13 or less, most preferably 12 or less). Further, a lower limit of the length of an amino acids sequence corresponding to the 131st to 139th amino acids sequence is preferably five or more.

Further, the protein for presenting a peptide of the present invention may be one wherein one to three amino acids are substituted in the first to 130th amino acids sequence or the 140th to 238th amino acids sequence in the amino acid sequence of SEQ ID NO: 54, and which exhibs a fluorescence generation activity. The protein consisting of the amino acid sequence of the SEQ ID NO: 54 is the sfGFP described in Non-patent literature 2. However, GFP-based proteins can be used as the scaffold protein for inserting the peptide in the protein for presenting a peptide of the present invention. That is to say, GFP or GFP-based variant protein derived from GFP can be used without limitation. As a concrete scaffold protein, there may be mentioned GFP, YFP, CFP, BFP, or RFP.

Preferably, the protein for presenting a peptide of the present invention can present a peptide in a presentation region consisting of the 131st to 138th amino acids in the amino acid sequence of the SEQ ID NO: 54, and the peptide consisting of four to fourteen amino acids (preferably 4 to 13 amino acids, more preferably 4 to 12 amino acids) is inserted in the 131st to 138th amino acids sequence, or substituted for any of 1 to 8 amino acid(s) sequence in the 131st to 138th amino acids sequence; and a total length of amino acids sequence corresponding to the 131st to 138th amino acids sequence is fourteen or less (preferably 13 or less, more preferably 12 or less). Further, a lower limit of the length of an amino acids sequence corresponding to the 131st to 138th amino acids sequence is preferably five or more. The protein for presenting a peptide of the present invention may be one wherein one to three amino acids are substituted in the first to 130th amino acids sequence or the 139th to 238th amino acids sequence in the amino acid sequence of SEQ ID NO:54, and which exhibits a fluorescence generation activity.

More preferably, the protein for presenting a peptide of the present invention can present a peptide in a presentation region consisting of the 131st to 137th amino acids in the amino acid sequence of the SEQ ID NO:54, and the peptide consisting of four to thirteen amino acids (preferably 4 to 12 amino acids) is inserted in the 131st to 137th amino acids sequence, or substituted for any of 1 to 7 amino acid(s) sequence in the 131st to 137th amino acids sequence; and a total length of amino acids sequence corresponding to the 131st to 137th amino acids sequence is thirteen or less (preferably 12 or less). Further, a lower limit of the length of an amino acids sequence corresponding to the 131st to 137th amino acids sequence is preferably five or more. The protein for presenting a peptide of the present invention may be one wherein one to three amino acids are substituted in the first to 130th amino acids sequence or the 139th to 238th amino acids sequence in the amino acid sequence of SEQ ID NO:54, and which exhibits a fluorescence generation activity.

The term "peptide-presenting protein" as used herein means, for example, a protein presenting a peptide in the region of the 131th to 139th amino acids of the sfGFP. For example, it means a sfGFP in which any peptides for peptide screening are previously inserted therein. As a concrete embodiment, it means a protein expressed from the vector included in the peptide library of the present invention.

On the other hand, the term "protein for presenting a peptide" as used herein means, for example, a protein which is designed so that a peptide can be presented in the region of the 131st to 139th amino acids of the sfGFP. For example, it means an sfGFP in which any peptides for peptide screening are not inserted yet, although any peptides for peptide screening can be inserted therein. As a concrete embodiment, it means a protein translated from the vector which is designed so that a peptide can be inserted in the region of the 131st to 139th amino acids of the sfGFP. Therefore, a polynucleotide encoding the protein for presenting a peptide preferably has restriction enzyme site(s) for inserting nucleotides encoding any peptides.

The region of the 131 st to 139th amino acids may be preferably a region the 131st to 138th amino acids, more preferably a region the 131st to 137th amino acids. This is because, when the length of the region is shorter, the peptide-presenting protein has a tendency to easily dissolve. Further, the length of the inserted peptide is preferably 4 to 15, more preferably 4 to 13, more preferably 4 to 13, most preferably 4 to 12. This is because, when the length of the peptide is shorter, the peptide-presenting protein has a tendency to easily dissolve.

### (Presentation region: 131st to 139th amino acids sequence)

A sequence of 131st to 139th amino acids in the amino acid sequence of sfGFP is KEDGNLGH (SEQ ID NO: 55). When the region of the nine amino acids sequence is substituted with a peptide consisting of 4 to 15 (preferably 4 to 14) amino acids sequence, a structural flexibility of the peptide is restricted so as to be presented on the sfGFP in a stable structure. Thus, peptide(s) highly specific to a target molecule can be screened by using the peptide-presenting proteins as the peptide library.

When the presentation region is the 131st to 139th amino acids sequence, the inserted or substituted amino acids sequence is 4 to 15 amino acids, preferably 4 to 14 amino acids, and may include a part of the 131st to 139th amino acids sequence. Thus, 4 to 6 amino acids sequence may be inserted without deletion of the 131st to 139th amino acids sequence. Further, any one of the number of 1 to 9 amino acids sequence in the 131st to 139th amino acids sequence are deleted, and a exogenous amino acid(s) sequence may be inserted so that the total number of the amino acids is 5 to 15, preferably 5 to 14.

Preferably, the presentation region is the 131st to 138th amino acids sequence. In this case, the inserted or substituted amino acids sequence is 4 to 14 amino acids, preferably 4 to 13 amino acids, and may include a part of the 131st to 138th amino acids sequence. Thus, 4 to 6 amino acids sequence may be inserted without deletion of the 131st to 138th amino acids sequence. Further, any one of the number of 1 to 8 amino acids sequence in the 131st to 138th amino acids sequence are deleted, and a exogenous amino acid(s) sequence may be inserted so that the total number of the amino acids is 4 to 14, preferably 4 to 13.

Further, in order to efficiently construct the peptide library, amino acid sequences of both ends of the inserted or substituted peptide is preferably one translated from base sequences containing restriction enzyme sites. The restriction enzyme sites for using for inserting the peptide is not limited. However, for example, BamHI, EcoRI, ScaI, SalI, SphI, PstI, NcoI, Nhel, SspI, NotI, SacI, PvuII, MfeI, HindIII, SbfI, EagI, EcoRV, AvrII, BstXI, PciI, HpaI, AgeI, BsmBI, BspQI, SapI, KpnI, or BsaI can be used. For example, the amino acid sequences of both ends of the inserted or substituted 4 to 14 peptides may be amino acid sequence translated from nucleotides containing the base sequence recognized by the above restriction enzyme.

In connection with this, the peptide translated from the restriction enzyme site is sometimes referred to as a linker peptide.

### (Deletion, substitution, insertion, and/or addition of amino acid(s))

As mentioned above, the GFP-based proteins can be used as the scaffold protein of the peptide-presenting protein and the protein for presenting a peptide, but the scaffold proteins may be proteins wherein one to three amino acids are substituted in the amino acid sequence encoding the GFP-based proteins, and the proteins exhibit a fluorescence generation activity.

The term "one to three amino acids are substitutedin the amino acid sequence" means the GFP-based protein itself or the GFP-based protein modified by the known methods such as site-directed mutagenesis, several naturally occurring amino acids substitution, or the like. The number of modified amino acids is 1 to 3, most preferably 1.

Preferably, examples of the modified amino acids sequence in the polypeptide according to the present invention may be an amino acids sequence having one to three conservative substitutions.

The term "conservative substitutions" used herein means that one to three amino acid residues contained in a protein are replaced with different amino acids having similar chemical properties. As for the conservative substitution, there may be mentioned, for example, a substitution of a hydrophobic residue for another hydrophobic residue, or a substitution of a polar residue for another polar residue having the same charge. Amino acids which have similar chemical properties and can be conservatively substituted with each other are known to those skilled in the art. More particularly, as for nonpolar (hydrophobic) amino acids, there may be mentioned, for example, alanine, valine, isoleucine, leucine, proline, tryptophan, phenylalanine, or methionine. As for polar (neutral) amino acids, there may be mentioned, for example, glycine, serine, threonine, tyrosine, glutamine, asparagine, or cysteine. As for basic amino acids having a positive charge, there may be mentioned, for example, arginine, histidine, or lysine. As for acidic amino acids having a negative charge, there may be mentioned, for example, aspartic acid or glutamic acid. In the "conservative substitution", an original amino acid may be substituted with an amino acid residue which is not chemically similar thereto, but structurally similar thereto. For example, it includes a substitution of alanine (A) for aspartic acid (D), a substitution of alanine (A) for asparagine (E), and a substitution of phenylalanine (F) for tyrosine (Y).

### (Fusion protein)

In the fusion protein of the present invention, the peptide-presenting protein, or the protein for presenting a peptide of the present invention is fused with a surface protein.

The term "surface protein" used herein means a protein which is presented on a surface of the host, as mentioned below. For example, when the host is a phage, it means a surface protein of the phage. Further, when the host is bacteria, it means a surface protein of the bacteria. Furthermore, when the host is yeast, it means a surface protein of the yeast; and when the host is animal cells, it means a surface protein of the animal cells.

The peptide-presenting protein can be presented on the surface of hosts, by fusing the peptide-presenting protein or protein for presenting a peptide with the surface protein of the hosts. Thus, when the peptide library of the present invention is subjected to an affinity selection (screening) of the peptide of interest, the peptide(s) of interest can be easily concentrated.

The surface protein of phage is not limited, but examples include g3p or g8p of M13 phage, G10 of T7 phage.

The surface protein of bacteria, for example Escherichia coli is not limited, but examples include OmpA.

The surface protein of yeast is not limited, but examples include Flo1, α agglutinin, or AGA2.

The surface protein of animal cells is not limited, but examples include human Toll-like receptor 4, EGFR, LDL receptor, angiotensin II receptor, or PDGFR.

### (Polynucleotide)

The polynucleotide of the present invention is one encoding the peptide-presenting protein of the present invention (hereinafter sometimes referred to as peptide-presenting polynucleotide), one encoding the protein for presenting a peptide of the present invention (hereinafter sometimes referred to as polynucleotide for presenting a peptide), or one encoding the fusion protein of the present invention (hereinafter sometimes referred to as fusion polynucleotide). The fusion polynucleotide may contain a nucleotide encoding a linker polypeptide which may exist between the peptide-presenting protein or protein for presenting a peptide, and the surface protein. In connection to this, the base sequence of the SEQ ID NO: 53 is a base sequence of nucleotide encoding the sfGFP.

### (Vector)

The polynucleotide of the present invention is not limited, but can be easily proliferated by being inserted into a vector. Further, it can be easily expressed as the peptide-presenting protein or the fusion protein.

The vector for inserting the polynucleotide is not limited, so long as it can be replicated in host cells. Any vectors such as plasmids, phages, or viruses can be used. For example, there may be mentioned a plasmid for Escherichia coli, such as pBR322, pBR325, pUC118, pUC119, pKC30, or pCFM536, a plasmid for Bacillus subtilis, such as pUB110, a plasmid for yeast, such as pG-1, YEp13, or YCp50, phage DNA such as λgt110, or λZAPII. Further, as the vector for mammalian cells, there may be mentioned a virus DNA such as baculovirus, vaccinia virus, or adenovirus; SV40; or derivatives thereof. The vector contains an initiation site of replication, a selective marker, and a promotor, and if necessary may contain an enhancer, a terminator, a ribosomal binding site, and polyadenylation signal.

For example, when the vector is used as the library of the present invention, M13 phage vector, or T7 phage vector is preferably used. This is because either can be used as a vector for phage display.

### (Host)

The "host" used herein is not limited as long as the vector can be inserted therein and the peptide-presenting protein or the fusion protein can be expressed thereby, and the host includes phages, bacteria, yeasts, insect cells, or animal cells. For example, as the host for the phage vector, there may be mentioned M13 phage, fd phage, or T7 phage.

Further, as the host for the bacteria vector, there may be mentioned Escherichia coli, Streptomyces, or Bacillus subtilis. Furthermore, as the host for the yeast vector, there may be mentioned baker's yeast, or methanol-assimilating yeast. As the host for insect cells, there may be mentioned Drosophila S2, or Spodoptera Sf9. Further, as the host for animal cells, there may be mentioned CHO, COS, BHK, 3T3, or C127.

### [2] Library

The library of the present invention is a library for peptide presentation, and the peptides presented thereon have random amino acid sequences. The library for peptide presentation can be used for screening peptide(s) capable of binding to a target molecule. Therefore, the presented peptides preferably contain a number of (many kinds of) peptides consisting of random amino acid sequences. Further, the library of the present invention contains the above host, and thus includes a phage display library, or a cell display library of bacteria, yeast, insect cells, or animal cells.

A length of the presented peptide provided for screening using the library of the present invention is not particularly limited, but for example, 4 to 15, preferably 4 to 14, more preferably 4 to 13.

### [3] Method for constructing library

The method for constructing a library comprises the steps of: introducing the vector mentioned above into a host, in particular (1) inserting a nucleotide encoding a peptide having 4 to 15 amino acids (preferably 4 to 14) into the vector containing polypeptide encoding the protein for presenting a peptide of the present invention to obtain an expression vector, wherein the peptide is inserted in the 131st to 139th amino acids sequence of the presentation region, or substituted for any one of 1 to 9 amino acid(s) sequence in the 131 st to 139th amino acids sequence of the presentation region; and a total length of amino acids sequence corresponding to the 131st to 139th amino acids sequence is 15 or less (preferably 14 or less), and (2) introducing the vector into a host. In particular, the library is the library for peptide presentation, and can present any peptides.

Preferably, the method for constructing a library comprises the steps of: (1) inserting a nucleotide encoding a peptide having 4 to 14 amino acids (preferably 4 to 13) into the vector containing polypeptide encoding the protein for presenting a peptide of the present invention to obtain an expression vector, wherein the peptide is inserted in the 131st to 138th amino acids sequence of the presentation region, or substituted for any one of 1 to 8 amino acid(s) sequence in the 131st to 138th amino acids sequence of the presentation region; and a total length of amino acids sequence corresponding to the 131st to 138th amino acids sequence is 14 or less (preferably 13 or less), and (2) introducing the vector into a host.

The vector used for the method for constructing a library contains polypeptide encoding the protein for presenting a peptide, and polynucleotides encoding any polypeptides can be easily inserted therein by using a restriction enzyme site.

A length of the polypeptide encoded by the inserted nucleotides is not particularly limited, but for example, 4 to 15, preferably 4 to 14, more preferably 4 to 13.

In the vector construction and the vector introduction into the host, the known methods can be used. For example, when the host is a phage, library genes of peptides to be presented are inserted into vector arms by a ligation. The resulting ligation solution is mixed with a packaging solution containing phage proteins to form phage particles. A phage library can be constructed by infected Escherichia coli such as BLT5403 with the phage particles.

### (Library gene)

Genes for inserting into a library can be prepared by known methods.

For example, a codon encoding any amino acids is substituted with NNK codons (wherein N is any one of A, G, C, or T, and K is any one of G or T) encoding random amino acids in a site-specific manner. The substitution is carried out by chemically synthesizing oligo DNAs containing the above region and inserting the same using a restriction enzyme; or using a method for replicating the full length vector using PCR.

### [4] Screening method

The screening method of the present invention comprises the steps of: (1) bringing into contact the above peptide library with the target molecule, and (2) recovering hosts binding to the target molecule. Preferably, the screening method further comprises (3) the step of proliferating the recovered hosts. In the screening method of the present invention, the contact step (1), preferably the host recovering step (2), and the proliferating step (3) are repeated, to thereby concentrate the peptides binding to a target molecule. The contact between the target molecule and the peptide library in the contact step (1) can be carried out by immobilizing the target molecule to an insoluble carrier such as wells or dishes.

The screening method of the present invention may be carried out according to known methods, except that the library of the present invention is used. For example, the screening for the phage library using phage as the host cell may be performed as follows. The phage library is brought into contact with the target molecule immobilized to a plate or the like so as to react. Phages which are not bound to the target molecule are removed by washing. Phages which are bound to the target molecule are recovered. Then, Escherichia Coli is infected with the phages and is lysed to thereby proliferate the phages. A supernatant after bacteriolysis or purified phages are further reacted with the immobilized target molecule to thereby concentrate the phages wherein a binding molecule specific to the target molecule is presented (Panning). Finally, the phages are cloned and the peptide presented thereon is determined by a sequence method.

The obtained peptide can bind with the target molecule. In the screening method of the present invention, as the peptides are presented on the protein for presenting a peptide, the structure of the peptides is stable. Therefore, the peptides highly specific to the target molecule can be screened.

### EXAMPLES

The present invention now will be further illustrated by, but is by no means limited to, the following Examples.

### <<Reference Example 1: Construction of sfGFP expression vector>>

In this reference example, a vector expressing sfGFP was constructed.

A PCR was carried out by a KOD FX (TOYOBO), using a pGEX-6P-3-PTD-3-ODD-EGFP vector (Kizaka-Kondoh et al., Clin. Cancer. Res., 2009, vol. 15, p.3433-3441) having a gene of Enhanced GFP (EGFP) as a template, and primers (SEQ ID NO: 1 and 2) (Table 2), to obtain a EGFP gene fragment of 0.7kb length in which restriction enzyme (Bam HI or Eco RI) sites were added to both ends. The fragment was digested with restriction enzymes Bam HI and EcoRI and it was ligated with a cloning pGEM-3Zf(+) vector (Promega) which was preliminarily digested with Bam HI and EcoRI. A pGEM-EGFP vector wherein the EGFP gene was inserted, was obtained.

A PCR was carried out, using the pGEM-EGFP vector as a template, primers (SEQ ID NO: 3 to 16) (Table 2), and PfuUltra High fidelity DNA Polymerase (Agilent Technologies) to obtain a pGEM-sfGFP vector wherein the sfGFP gene which has site-directed mutations for substituting 9 amino acids was inserted. The vector was digested with Bam HI and Eco RI to obtain a Bam HI-Eco RI fragment. The fragment was bound to a BamHI and Eco RI site of a pGEX-6P-3 vector for expression of GST fusion protein (GE Healthcare) to obtain a pGEX-sfGFP vector. The pGEX-sfGFP has a length of 5.6kbp, and has in order of a base sequence of GST structural gene and a base sequence of sfGFP at the downstream of tac promoter. Therefore, the pGEX-sfGFP vector intracellularly expresses a fusion protein containing in order of GST and sfGFP in the Escherichia Coli.

In these examples, peptide-presenting proteins wherein a caspase-3 recognizing peptide was inserted in the 131st to 139th amino acids sequence, were prepared.

A PCR was carried out using the pGEX-sfGFP vector constructed in the reference example 1 as a template, primers (SEQ ID NO: 7, 18, 23-28, 31-46, 58-59, and 64-71) (Table 2), and PfuUltra High fidelity DNA Polymerase to obtain a pGEX-m1 vector (Example 1), pGEX-m4 to pGEX-m6 vectors (Examples 2 to 4), pGEX-m10 to pGEX-m22 vectors (Examples 5 to 17) for expressing sfGFP in which the caspase-3 recognizing peptide (DEVD) is inserted in a site-specific manner. Lengths and positions of substituted peptides are shown in Table 1. The peptide-presenting proteins obtained in Example 16 (m10) and Example 17 (m22) were easily insolubilized.

### <<Comparative Examples 1 to 5>>

In these comparative examples, peptide-presenting proteins wherein the caspase-3 recognizing peptide was inserted in an amino acid sequence other than the 131st to 139th amino acids sequence, were prepared.

The procedure described in Example 1 was repeated except that primers having base sequences of SEQ ID NO: 19 and 20 (Comparative Example 1), SEQ ID NO: 21 and 22 (Comparative Example 2), SEQ ID NO: 29 and 30 (Comparative Example 4), SEQ ID NO: 60 and 61 (Comparative Example 4), and SEQ ID NO: 62 and 63 (Comparative Example 5) were used, to obtain a pGEX-m2 vector (Comparative Example 1), a pGEX-m3(Comparative Example 2), a pGEX-m7(Comparative Example 3), a pGEX-m8(Comparative Example 4), and a pGEX-m9(Comparative Example 5). Lengths and positions of substituted peptides are shown in Table 1.

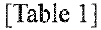

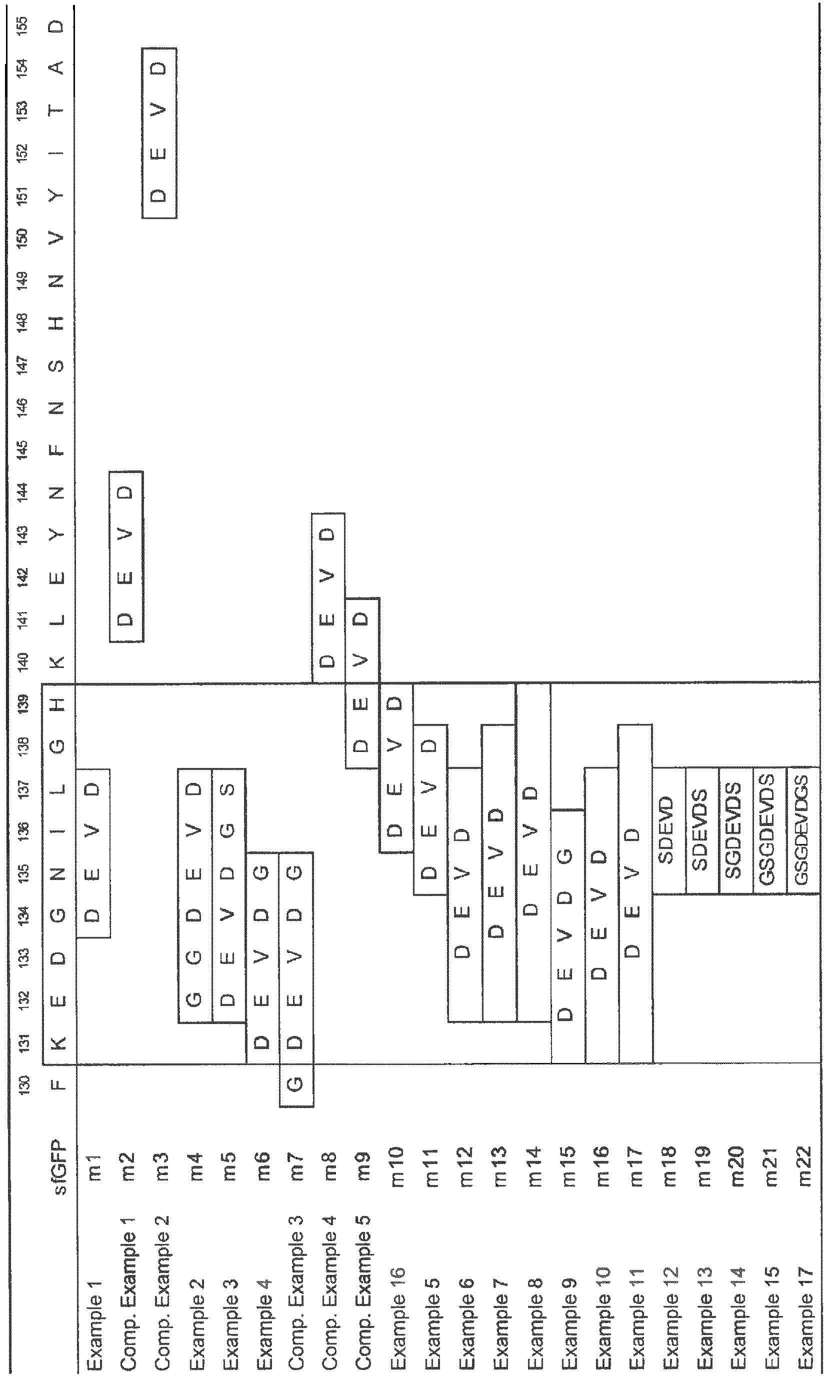

### «Expression and purification of peptide-presenting protein»

Peptide-presenting proteins were expressed and purified using the vectors obtained in Examples 1 to 17, and Comparative Examples 1 to 5.

An Escherichia Coli BL21-CodonPlus (DE3) strain (Agilent Technologies) was transformed with the respective pGEX-sfGFP vectors, i.e. pGEX-m1 to pGEX-m22 vectors by a heat shock method. Each of the Escherichia Coli containing the expression vectors was cultured in a 50 mL of LBA medium (containing 0.01% ampicillin, 1%Bacto Tryptone, 0.5%Bacto Yeast extract, 1%NaCl) at 37°C, so as to be 0.5 of O.D.600. Then, Isopropyl β-D-1-thiogalactopyranoside was added to the medium to give 0.1mM of the final concentration, and the Escherichia Coli was cultured at 20°C over night, to obtain an Escherichia Coli wherein a GST fusion protein was expressed.

Each of the Escherichia Coli in which the protein was expressed was collected by centrifugation and was re-suspended in 20mL of phosphate buffer solution (PBS). The obtained bacteria were crushed by three freeze-thaw, and ultrasonic fragmentation, Subsequently, Triton x-100 was added thereto to give 1% of the final concentration, and the whole was allowed to stand on ice for 30 minutes. Next, the solution containing bacteria were centrifuged at 5000 rpm for 10 minutes at 4°C, and the supernatant containing the protein was collected. Then, the collected solution was added to GST agarose beads washed by PBS, and the GST fusion protein was adsorbed thereto while gently rotating at 4°C over night. The agarose beads were washed by PBS, and then washed by Prescission Cleavage Buffer (0.05M Tris-HCl(pH7.0), 0.15M NaCl, 1mM EDTA, 1mM DTT). Subsequently, in order to cleave the fusion protein to GST and sfGFP or sfGFP variants (sfGFP-ml to sfGFP-m22), 1mL of Prescission Cleavage Buffer and 5µL of Prescission Protease (GE Healthcare) were added thereto. The whole was reacted at room temperature for 4 hours, and then reacted at 4°C over night. Next, the reactant was centrifuged at 3000rpm for 15 minutes at 4°C, to collect a supernatant. A buffer of the supernatant was substituted by an ultrafiltration membrane capable of cutting 10kDa, to obtain about 5mg of peptide-presenting protein (sfGFP-ml to m9 and sfGFP-m11 to m21) (hereinafter, the peptide-presenting proteins obtained from pGEX-m1 to m9 and pGEX-m11 to m21 are sometimes referred to as m1 to m9 and m11 to m21, respectively). The sfGFP-m10 and sfGFP-m22 were expressed in inclusion bodies.

### «Cleavage of peptide-presenting protein by caspase-3 »

In the peptide-presenting proteins obtained from pGEX-m1 to m9 and m11 to m21, the caspase-3 recognizing peptide (DEVD) was inserted. In order to study the structural flexibility of the peptide-presenting protein, the peptide-presenting proteins were cleaved by caspase-3.

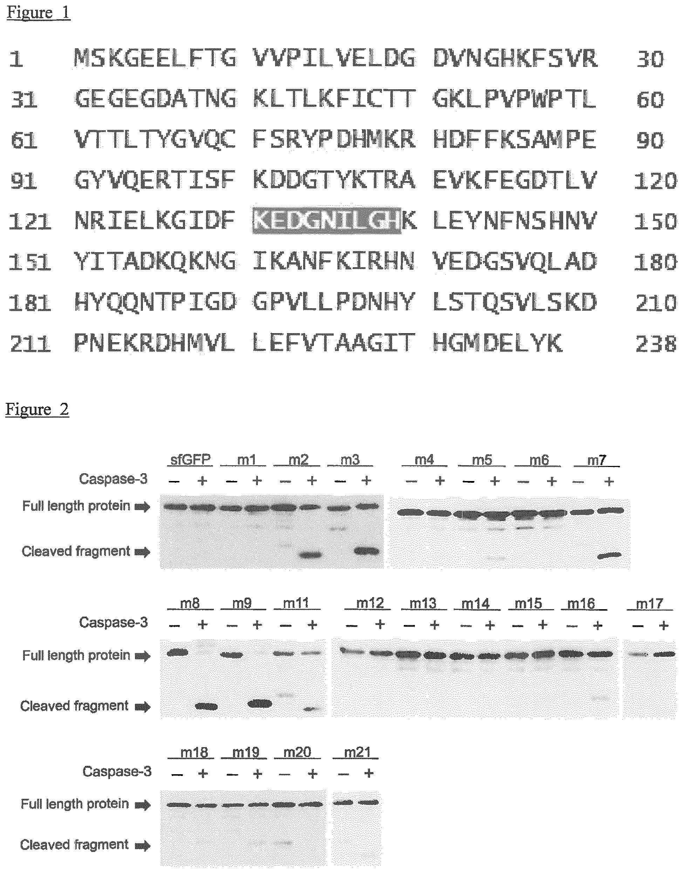

10µM of the peptide-presenting proteins m1 to m9 and m11 to m21were reacted with 4mU/mL of human caspase-3 (Sigma-Aldrich) at 37°C for 24 hours in a caspase reaction buffer (20mM pipes(pH7.2), 100mM NaCl, 0.1% CHAPS, 10% sucrose, 10mM DTT). The sfGFP was used as a control. The reacted solutions were subjected to a 15% acrylamide gel electrophoresis, and transferred to a Hybond ECL membrane (GE Healthcare) at 20V constant voltage for 1 hour. Next, after blocking with a 5% skim milk, the membrane was reacted with a first antibody (rabbit polyclonal anti-GFP antibody) at 4°C over night. Then, the membrane was reacted with a second antibody (HRP conjugated anti-rabbit IgG antibody) at room temperature for 1 hour, and cleaved fragments were detected by adding ECL prime (GE Healthcare). As a result, m2 (Comparative Example 1), m3 (Comparative Example 2), m7 (Comparative Example 3), m8 (Comparative Example 4), and m9 (Comparative Example 5) were cleaved by caspase-3. On the other hand, m1 (Example 1), m4 (Example 2), m5 (Example 3), m6 (Example 4), m11 (Example 5), m12 (Example 6), m13 (Example 7), m14 (Example 8), m15 (Example 9), m16 (Example 10), m17 (Example 11), m18 (Example 12), m19 (Example 13), m20 (Example 14), and m21 (Example 15) were hardly cleaved (Table 1 and Figure 2). These results show that when the caspase-3 recognizing peptides having amino acid lengths of 5 to 14 were inserted in the region of the 131st to 139th amino acids of the sfGFP , they could not bind to caspase-3, and when the caspase-3 recognizing peptides were inserted in regions other than the above region, they could bind to caspase-3 That is to say, the peptides inserted in the region of the 131 st to 139th amino acids were structurally fixed, and it is considered that the structural flexibility thereof was limited.

### «Fluorescence intensity of peptide-presenting protein»

An influence on a fluorescence intensity of peptide-presenting protein due to the insertion of the peptides was investigated.

The peptide-presenting proteins (m1 to m9 and m11 to m21) were dissolved in a buffer (20mM Pipes(pH7.2), 100mM NaCl, 0.1%CHAPS, 10 sucrose, 10mM DTT) to give 10µM of the concentration, and the fluorescence (excitation wavelength / fluorescence wavelength=480/510nm) was measured. The fluorescence was observed in the peptide-presenting proteins of m1 (Example 1), m4 (Example 2), m5 (Example 3), m6 (Example 4), m11 (Example 5), m12 (Example 6), m13 (Example 7), m14 (Example 8), m15 (Example 9), m16 (Example 10), m17 (Example 11), m18 (Example 12), m19 (Example 13), m20 (Example 14), and m21 (Example 15) wherein the structural flexibility of the peptides was limited, but the fluorescence was quenched in the peptide-presenting proteins of m2 (Comparative Example 1), m3 (Comparative Example 2), m7 (Comparative Example 3), m8 (Comparative Example 4), m9 (Comparative Example 5) (Table 1 and Table 3). That is, the results showed that the fluorescence of GFP was maintained, when the caspase-3 recognizing peptides having amino acids length of 5 to 14 were inserted in the region of the 131st to 139th amino acids of the sfGFP.

### «Analytical Example 1: Analysis of conformational fluctuation of K131-H139 region»

If the structural flexibility of the peptides inserted into the prepared peptide-presenting protein was limited, it was considered that a conformational fluctuation of the K131-H139 region in a molecular dynamics simulation was reduced.

The molecular dynamics simulations of sfGFP, m1 (Example 1), m6 (Example 4), and m19 (Example 13), which were not easily cleaved by caspase-3, were carried out. As a control, the m7 (Comparative Example 3) which was cleaved by caspase-3, was used. Initial model of these proteins were prepared from a crystallographic structure of sfGFP (PDB ID:2B3P). For simplicity of calculation, two amino acids (65T and 66Y) which constitute fluorophore were substituted to glycine residues. Thermodynamically stable structures thereof were calculated from the initial model using a force field of Amber 11 program package (Amber). In the molecular dynamics simulation, a structure for 10 nanoseconds in a GB/SA solvent model was calculated using a Sander module of Amber 11, and then the fluctuation was evaluated by using a structure of 4.5 to 10 nanoseconds which reaches a state of equilibrium.

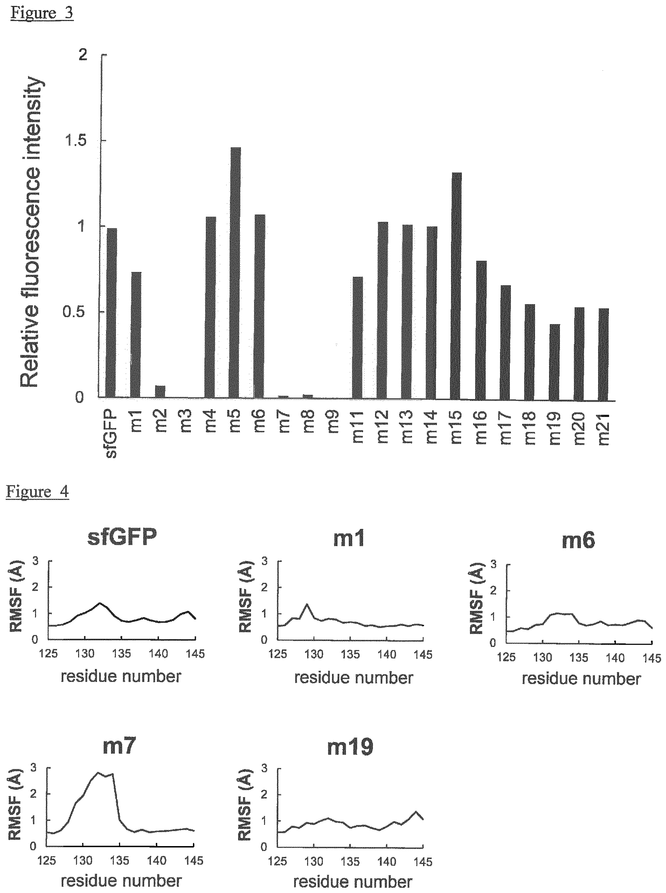

The results are shown in Figure 4. A longitudinal axis is a magnitude of fluctuation of each residue at 4.5 to 10 nanoseconds in the simulation, and an abscissa axis is the residue number. The fluctuations in the K131-H139 region of the sfGFP, m1 (Example 1), m6 (Example 4), m19 (Example 13) were within 0.1 nanometers at the most residues. However, the fluctuations in the K131-H139 region of the m7(Comparative Example 3) was increased up to about 0.3 nanometers, and the results showed that the K131-H139 region may fluctuate widely. Thus, it was revealed that the variants which were not easily cleaved by caspase-3 have little fluctuation, and the structural flexibility of the peptide inserted therein was limited.

### <<EXAMPLES 16 to 20>>

In this example, five peptides which bind to a breast cancer marker protein, i.e. HER2 according to reports were inserted in the 131st to 139th amino acids sequence region of sfGFP, to obtain expression vectors and purified proteins.

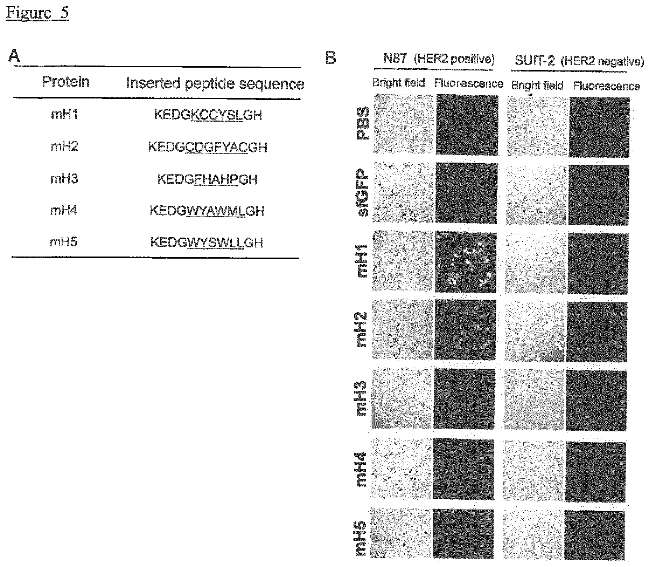

The procedures described in Example 1 were repeated except that primers of SEQ ID NO: 47 and 48 (Example 16), SEQ ID NO: 49 and 50 (Example 17), SEQ ID NO: 51 and 52 (Example 18), SEQ ID NO: 72 and 73 (Example 19), or SEQ ID NO: 74 and 75 (Example 20) used as primers, to obtain pGEX-mH1 vector (Example 16), pGEX-mH2vector (Example 17), pGEX-mH3vector (Example 18), pGEX-mH4vector (Example 19), or pGEX-mH5vector (Example 20). Lengths and positions of substituted peptides are shown in Figure 5(A). A sequence of mH1 is "KEDGKCCYSLGH" (SEQ ID NO:55), a sequence of mH2 is "KEDGCDGFYACGH" (SEQ ID NO:56), a sequence of mH3 is "KEDGFHAHPGH"(SEQ ID NO:57), a sequence of mH4 is "KEDGWYAWMLGH" (SEQ ID NO:76), and a sequence of mH is "KEDGWYSWLLGH" (SEQ ID NO:77).

Further, the procedures described in the item of "Expression and purification of peptide-presenting protein" were repeated except that the pGEX-mH1 vector, pGEX-mH2 vector, pGEX-mH3 vector, pGEX-mH4 vector, and pGEX-mH5 vector were used to obtain peptide-presenting proteins (hereinafter, the peptide-presenting proteins obtained from the pGEX-mH1 to mH5 vectors are sometimes referred to as mH1 to mH5, respectively).

The HER2 protein which may frequently be overexpressed in breast cancer cells, is useful for a target for treatment or imaging of breast cancer, and a peptide which can function in a living body of a mouse was reported. The peptide, mH1, was screened from a phage library, and has an S-S bond between neighboring cysteines, and the construction thereof was partially limited (Karasseva et al., J. Protein Chem., 2002, vol.21, p.287-296). The peptide, mH2, was designed on the basis of a binding surface of an antibody binding to HER2, and exhibits the binding activity under the condition that the peptide is circularized and the structure thereof is limited (Park et al., Nat. Biotechnol., 2000, vol.18, p.194-198). The peptide, mH3, was selected by in silico screening from an unstructured peptide librariy (Nakajima et al., Breast Cancer, 2008, vol.15, p.65-72). The peptides, mH4 and mH5, were screened concomitantly with the peptide, mH1, and the structures thereof were not limited. It was preliminarily reported that the mH1 and mH2 can function in a living body of a mouse (Deutscher SL, Chem. Rev., 2010, vol.110, p.3196-3211).

### «Binding to cancer cells, of peptide-presenting protein wherein the HER2 peptide is inserted»

2.5x10⁴ of HER2 positive N87 stomach cancer cell or HER2 negative SUIT-2 pancreatic cancer cell was cultured in 8well slide chamber culture plate for 16 hours. The cells were fixed by being reacted with a 4% paraformaldehyde phosphate buffer for 15 minutes, washed by PBS solution, and blocked using 3% bovine serum albumin/TBST for 15 minutes. Then, after adding 2µM of peptide-presenting protein (mH1, mH2, mH3, mH4, or mH5), the cells were incubated at 4°C for 16 hours, and binding activities were evaluated by a fluorescence microscope observation. As a result, it was revealed that mH1 and mH2 specifically bind to N87 cell, but mH3, mH4, and mH5 may not bind thereto (Figure 4(B)). That is, when the peptides which were screened under the conditions that the structures thereof were not limited, were inserted into the vector, the peptides exhibit a high binding property as well. However, when the peptides which were screened under the conditions that the structure of peptides can freely vary, the peptides cannot bind thereto. It was already known that the former can function in a living body of a mouse. Therefore, the results showed that the peptides which have high specificity to target and function in a living body, can be easily and high-sensitively screened by using the protein wherein the peptides were inserted in the K131- H139 region of sfGFP, and evaluating the same.

**[Table 2-1]**

| | |
|---|---|
| SEQ ID No: 1 : | 5'-TCTAGAGGATCCATGGTGAGCAAGGGCGAGGAG-3' |
| SEQ ID No: 2 : | 5'-TAGGGCGAATTCTTAATGGTGATGGTGATGATGGCTGCCCTTGTACAGCTCGTCCATGCC-3' |
| SEQ ID No: 3 : | 5'-CACAAGTTCAGCGTGCGCGGCGAGGGCGAGGGCGATGCCACCAACGGCAAGCTGACCCTG-3' |
| SEQ ID No: 4 : | 5'-CAGGGTCAGCTTGCCGTTGGTGGCATCGCCCTCGCCCTCGCCGCGCACGCTGAACTTGTG-3' |
| SEQ ID No: 5 : | 5'-CAGGAGCGCACCATCAGCTTCAAGGACGACGGCACCTACAAGACCCGCGCC-3' |
| SEQ ID No: 6 : | 5'-GGCGCGGGTCTTGTAGGTGCCGTCGTCCTTGAAGCTGATGGTGCGCTCCTG-3' |
| SEQ ID No: 7: | 5'-CTGAGCACCCAGTCCGTGCTGAGCAAAGACCCC-3' |
| SEQ ID No: 8 : | 5'-GGGGTCTTTGCTCAGCACGGACTGGGTGCTCAG-3' |
| SEQ ID No: 9 : | 5'-CAAGCTGGAGTACAACTTCAACAGCCACAACGTC-3' |
| SEQ ID No: 10 : | 5'-GACGTTGTGGCTGTTGAAGTTGTACTCCAGCTTG-3' |
| SEQ ID No: 11 : | 5'-CACAACGTCTATATCACCGCCGACAAGCAGAAG-3' |
| SEQ ID No: 12 : | 5'-CTTCTGCTTGTCGGCGGTGATATAGACGTTGTG-3' |
| SEQ ID No: 13 : | 5'-GAAGAACGGCATCAAGGCGAACTTCAAGATCCGC-3' |
| SEQ ID No: 14 : | 5'-GCGGATCTTGAAGTTCGCCTTGATGCCGTTCTTC-3' |
| SEQ ID No: 15 : | 5'-CAAGATCCGCCACAACGTGGAGGACGGCAGCGTG-3' |
| SEQ ID No: 16 : | 5'-CACGCTGCCGTCCTCCACGTTGTGGCGGATCTTG-3' |
| SEQ ID No: 17 (m1, Example 1) : | 5'-GATGAAGTTGATGAGTACAACTTCAACAGC-3' |
| SEQ ID No: 18 (m1, Example 1) : | 5'-CAGGATGTTGCCGTCCTC-3' |
| SEQ ID No: 19 (m2, Comp. Example 1) : | 5'-AACATCCTGGGGCACAAGGATGAGGTTGATTTCAACAGCCACAACGTC-3' |
| SEQ ID No: 20 (m2, Comp. Example 1) : | 5'-ACGTTGTGGCTGTTGAAATCAACCTCATCCTTGTGCCCCAGGATGTT-3' |
| SEQ ID No: 21 (m3, Comp. Example 2): | 5'-GATGAAGTTGATGACAAGCAGAAGAACGGC-3' |
| SEQ ID No: 22 (m3, Comp. Example 2) : | 5'-GACGTTGTGGCTGTTGAA-3' |
| SEQ ID No: 23 (m4, Example 2) : | 5'-GGCGGCGATGAAGTTGATGGGCACAAGCTGGAGTAC-3' |
| SEQ ID No: 24 (m4, Example 2) : | 5-CTTGAAGTCGATGCCCTT-3' |
| SEQ ID No: 25 (m5, Example 3) : | 5'-GATGAAGTTGATGGCAGCGGGCACAAGCTGGAGTAC-3' |
| SEQ ID No: 26 (m5, Example 3) : | 5'-CTTGAAGTCGATGCCCTT-3' |
| SEQ ID No: 27 (m6, Example 4) : | 5'-GATGAAGTTGATGGCATC-3' |
| SEQ ID No: 28 (m6, Example 4) : | 5'-GAAGTCGATGCCCTTCAG-3' |
| SEQ ID No: 29 (m7, Comp. Example 3) : | 5-GGCGATGAAGTTGATGGCATCCTGGGGCACAAGCTG-3' |
| SEQ ID No: 30 (m7, Comp. Example 3) : | 5'-GTCGATGCCCTTCAGCTC-3' |
| SEQ ID No: 31 (m12, Example 6) : | 5'-GATGAAGTTGATGGGCACAAGCTGGAGTAC-3' |
| SEQ ID No: 32 (m12, Example 6) : | 5'-CTTGAAGTCGATGCCCTT-3' |
| SEQ ID No: 33 (m13, Example 7) : | 5'-CACAAGCTGGAGTACAAC-3' |
| SEQ ID No: 34 (m13, Example 7) : | 5'-ATCAACTTCATCCTTGAAGTCGATGCC-3' |
| SEQ ID No: 35 (m14, Example 8) : | 5'-AAGCTGGAGTACAACTTC-3' |
| SEQ ID No: 36 (m14, Example 8) : | 5'-ATCAACTTCATCCTTGAAGTCGATGCC-3' |
| SEQ ID No: 37 (m15, Example 9) : | 5'-GGCGATGAAGTTGATGGCCTGGGGCACAAGCTG-3' |
| SEQ ID No: 38 (m15, Example 9) : | 5'-GAAGTCGATGCCCTTCAG-3' |

**[Table 2-2]**

| | |
|---|---|
| SEQ ID No: 39 (m16, Example 10) : | 5'-GATGAAGTTGATGGGCACAAGCTGGAGTAC-3 |
| SEQ ID No: 40 (m16, Example 10) : | 5'-GAAGTCGATGCCCTTCAG-3' |
| SEQ ID No: 41 (m18, Example 12) : | 5'-TCCGACGAGGTGGACGGGCACAAGCTGGAGTAC-3' |
| SEQ ID No: 42 (m18, Example 12) : | 5'-GCCGTCCTCCTTGAAGTCGAT-3' |
| SEQ ID No: 43 (m19, Example 13) : | 5'-TCCGACGAGGTGGACTCCGGGCACAAGCTGGAGTAC-3' |
| SEQ ID No: 44 (m19, Example 13) : | 5-GCCGTCCTCCTTGAAGTCGAT-3' |
| SEQ ID No: 45 (m20, Example 14) : | 5'-TCCGGGGACGAGGTGGACTCCGGGCACAAGCTGGAGTAC-3' |
| SEQ ID No: 46 (m20, Example 14) : | 5'-GCCGTCCTCCTTGAAGTCGAT-3' |
| SEQ ID No: 47 (mH1, Example 16) : | 5'-CCGGCGAGTCCGGCGGATCGAGAAGTGGGGCACAAGCTGGAG-3' |
| SEQ ID No: 48 (mH1, Example 16) : | 5'-GCCGTCCTCCTTGAAG-3' |
| SEQ ID No: 49 (mH2, Example 17) : | 5'-CTTCAAGGAGGACGGCTGCGATGGTTTTTATGCGTGCGGGCACAAGCTGGAG-3' |
| SEQ ID No: 50 (mH2, Example 17) : | 5'-CTCCAGCTTGTGCCCGCACGCATAAAAACCATCGCAGCCGTCCTCCTTGAAG-3' |
| SEQ ID No: 51 (mH3, Example 18) : | 5'-AAATGCTGCTATAGTCTGGGGCACAAGCTGGAG-3' |
| SEQ ID No: 52 (mH3, Example 18) : | 5-GCCGTCCTCCTTGAAG-3' |
| SEQ ID No: 58 (m22, Example 17) : | 5'-GGGTCCGGGGACGAGGTGGACGGGTCCGGGCACAAGCTGGAGTAC-3' |
| SEQ ID No: 59 (m22, Example 17) : | 5'-GCCGTCCTCCTTGAAGTCGAT-3' |
| SEQ ID No: 60 (m8, Comp. Example 4) : | 5'-AACATCCTGGGGCACGATGAGGTTGATAACTTCAACAGCCACAACGTC-3' |
| SEQ ID No: 61 (m8, Comp. Example 4) : | 5'-GACGTTGTGGCTGTTGAAGTTATCAACCTCATCGTGCCCCAGGATGTT-3' |
| SEQ ID No: 62 (m9, Comp. Example 5) : | 5'-GAGTACAACTTCAACAGC-3' |
| SEQ ID No: 63 (m9, Comp. Example 5) : | 5'-ATCAACTTCATCCAGGATGTTGCCGTCCTC-3' |
| SEQ ID No: 64 (m10, Example 16) : | 5'-GTTGATAAGCTGGAGTACAAC-3' |
| SEQ ID No: 65 (m10, Example 16) : | 5'-TTCATCGTTGCCGTCCTCCTT-3' |
| SEQ ID No: 66 (m11, Example 5) : | 5'-GTTGATCACAAGCTGGAGTAC-3' |
| SEQ ID No: 67 (m11, Example 5) : | 5'-TTCATCGCCGTCCTCCTTGAA-3' |
| SEQ ID No: 68 (m17, Example 11) : | 5'-AAGGGCATCGACTTCGATGAAGTTGAT-3' |
| SEQ ID No: 69 (m17, Example 11) : | 5'-ATCAACTTCATCGAAGTCGATGCCCTT-3' |
| SEQ ID No: 70 (m21, Example 15) : | 5'-GGGGACGAGGTGGACTCCGGGCACAAGCTG-3' |
| SEQ ID No: 71 (m21, Example 15) : | 5'-CAGCTTGTGCCCGGAGTCCACCTCGTCCCC-3' |
| SEQ ID No: 72 (mH4, Example 19) : | 5'-TGGTACAGCTGGCTGCTGGGGCACAAGCTGGAGTAC-3' |
| SEQ ID No: 73 (mH4, Example 19) : | 5'-GCCGTCCTCCTTGAAGTC-3' |
| SEQ ID No: 74 (mH5, Example 20) : | 5'-TGGTACGCCTGGATGCTHGGGCACAAGCTGGAGTAC-3' |
| SEQ ID No: 75 (mH5, Example 20) : | 5'-GCCGTCCTCCTTGAAGTC-3' |
| SEQ ID No: 76 : | KEDGWYAWMLGH |
| SEQ ID No: 77 : | KEDGWYSWLLGH |

### << Analytical Example 2: Analysis of conformational fluctuation of K131-L137 region>>

The molecular dynamics simulations of m12 (Example 6), m15 (Example 9), m16 (Example 10), m19 (Example 13), m21 (Example 15), and m22 (Example 17), which were peptide-presenting proteins having a K131-L137 region of the present invention, were carried out. The sfGFP was used as a positive control. The procedures described in analytical example 1 were repeated except that the m12 (Example 6), m15 (Example 9), m16 (Example 10), m19 (Example 13), m21 (Example 15), and m22 (Example 17) were used.

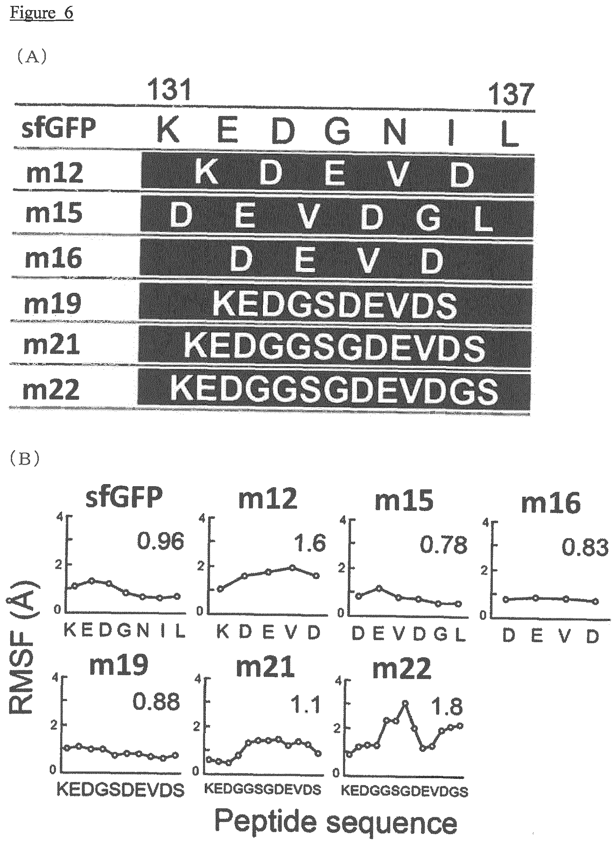

The amino acid sequences are shown in Figure 6(A) and the analysis results are shown in Figure 6(B). A longitudinal axis is a magnitude of fluctuation of each residue at 4.5 to 10 nanoseconds in the simulation, and an abscissa axis is the residue number. It was revealed that the variants which were not easily cleaved by caspase-3 have little fluctuation, and the structural flexibility of the peptide inserted therein was limited.

### INDUSTRIAL APPLICABILITY

The peptide-presenting protein and the peptide library using the same can be used as a screening of peptides capable of specifically binding to a target molecule. The peptides screened by using the peptide library of the present invention can be effectively used as an active ingredient for the medicament.

Although the present invention has been described with reference to specific embodiments, various changes and modifications obvious to those skilled in the art are possible without departing from the scope of the appended claims.

## Claims

1. A peptide-presenting protein wherein a peptide is presented in a presentation region consisting of the 131^{st} to 139^{th} amino acids in
(1) the amino acid sequence of the SEQ ID NO:54 or
(2) the amino acid sequence of SEQ ID NO:54, wherein one to three amino acids are substituted in the first to 130^{th} amino acids sequence or the 140^{th} to 238^{th} amino acids sequence, wherein the peptide consisting of four to fifteen amino acids is inserted in the 131^{st} to 139^{th} amino acids sequence, or substituted for any of 1 to 9 amino acid(s) sequence in the 131^{st} to 139^{th} amino acids sequence; and a total length of an amino acid sequence corresponding to the 131^{st} to 139^{th} amino acids sequence is fifteen or less, and
the protein exhibits a fluorescence generation activity.

2. A fusion protein wherein the peptide-presenting protein according to claim 1, and a surface protein are fused.

3. A polynucleotide encoding the peptide-presenting protein according to claim 1, or the fusion protein according to claim 2.

4. A vector comprising the polynucleotide according to claim 3.

5. A host comprising the vector according to claim 4.

6. A library comprising the host according to claim 5, wherein the presented peptides are a number of peptides consisting of random amino acid sequences.

7. A method for constructing a library, comprising the steps of:
(1) inserting a polynucleotide encoding a peptide consisting of 4 to 15 amino acids into a vector comprising a polynucleotide encoding
a protein for presenting a peptide or
a fusion protein wherein the protein for presenting a peptide is fused with a surface protein,
to obtain an expression vector, wherein the peptide is inserted into the 131st to the 139th amino acids sequence of the presentation region, or substituted for any of 1 to 9 amino acids sequence in the 131st to 139th amino acids sequence of the presentation region; and a total length of amino acids sequence corresponding to the 131st to 139th amino acid sequence is fifteen or less, and
(2) introducing the vector into a host,
wherein the protein for presenting a peptide presents the peptide in a presentation region consisting of the 131st to 139th amino acids in the amino acid sequence of the SEQ ID NO.54, wherein the peptide consisting of four to fifteen amino acids is inserted in the 131st to 139th amino acids sequence, or substituted for any of 1 to 9 amino acids(s) sequence corresponding to the 131st to 139th amino acids sequence; and a total length of amino acids sequence corresponding to the 131st to 139th amino acids sequence is fifteen or less.

8. A method for screening a peptide binding to a target molecule, comprising the steps of:
(1) bringing into contact the peptide library according to claim 6 with the target molecule,
(2) recovering hosts binding to the target molecule.

9. The method for screening a peptide according to claim 8, further comprising (3) the step of proliferating the recovered hosts.

10. The method for screening a peptide according to claim 9, wherein the contact step (1), the host recovering step (2), and the proliferating step (3) are repeated.

## Patentansprüche

1. Ein Peptid-präsentierendes Protein, wobei ein Peptid in einer Präsentationsregion, die aus der 131sten bis 139sten Aminosäure in
(1) der Aminosäuresequenz der SEQ ID NO:54 oder
(2) der Aminosäuresequenz der SEQ ID NO:54, wobei eine bis drei Aminosäuren der ersten bis 130sten Aminosäuren der Sequenz oder der 140sten bis 238sten Aminosäuren der Sequenz ersetzt sind, besteht, präsentiert wird,
wobei das aus vier bis fünfzehn Aminosäuren bestehende Peptid in die Abfolge der 131sten bis 139sten Aminosäure insertiert wird, oder eine beliebige Abfolge von der 1 bis 9 Aminosäuren in der Abfolge der 131sten bis 139sten Aminosäure durch dieses ersetzt wird; und die Gesamtlänge der Aminosäuresequenz, die der Abfolge der 131sten bis 139sten Aminosäure entspricht, fünfzehn oder weniger beträgt, und das Protein eine Fluoreszenzgenerierende Aktivität aufweist.

2. Ein Fusionsprotein, wobei das Peptid-präsentierende Protein gemäß Anspruch 1 und ein Oberflächenprotein fusioniert sind.

3. Ein Polynukleotid, das das Peptid-präsentierende Protein gemäß Anspruch 1 oder das Fusionsprotein gemäß Anspruch 2 kodiert.

4. Ein Vektor, der das Polynukleotid gemäß Anspruch 3 umfasst.

5. Ein Wirt, der den Vektor gemäß Anspruch 4 umfasst.

6. Eine Bibliothek, die den Wirt gemäß Anspruch 5 umfasst, wobei die präsentierten Peptide eine Anzahl von Peptiden sind, die aus zufälligen Aminosäuresequenzen bestehen.

7. Ein Verfahren zur Erstellung einer Bibliothek, das die Schritte umfasst:
(1) Insertion eines Polynukleotids, das ein aus 4 bis 15 Aminosäuren bestehendes Peptid kodiert, in einen Vektor, der ein Polynukleotid umfasst, das ein Protein zur Präsentation eines Peptids oder ein Fusionsprotein, wobei das Protein zur Präsentation eines Peptids mit einem Oberflächenprotein fusioniert ist, kodiert,
um einen Expressionsvektor zur erhalten, wobei das Peptid in die Abfolge der 131sten bis 139sten Aminosäure der Präsentationsregion insertiert wird, oder eine beliebige Abfolge von 1 bis 9 Aminosäuren der Abfolge der 131sten bis 139sten Aminosäure der Präsentationsregion ersetzt wird; und die Gesamtlänge der Aminosäuresequenz, die der Abfolge der 131sten bis 139sten Aminosäure entspricht, fünfzehn oder weniger beträgt, und
(2) Einführen des Vektors in einen Wirt,
wobei das Protein zur Präsentation eines Peptids das Peptid in einer Präsentationsregion präsentiert, die aus der 131sten bis 139sten Aminosäure in der Aminosäuresequenz der SEQ ID NO:54 besteht, wobei das aus vier bis fünfzehn Aminosäuren bestehende Peptid in die Abfolge der 131sten bis 139sten Aminosäure insertiert wird, oder eine beliebige Abfolge von 1 bis 9 Aminosäuren, die der Abfolge der 131sten bis 139sten Aminosäure entsprechen, ersetzt wird; und die Gesamtlänge der Aminosäuresequenz, die der Abfolge der 131sten bis 139sten Aminosäure entspricht, fünfzehn oder weniger beträgt.

8. Ein Verfahren zum Screening eines an ein Zielmolekül bindenden Peptids, das die Schritte umfasst:
(1) Das in Kontakt Bringen der Peptidbibliothek gemäß Anspruch 6 mit dem Zielmolekül,
(2) die Rückgewinnung der an das Zielmolekül bindenden Wirte.

9. Das Verfahren zum Screening eines Peptids gemäß Anspruch 8, das darüber hinaus (3) den Schritt der Vermehrung der wiedergewonnenen Wirte umfasst.

10. Das Verfahren zum Screening eines Peptids gemäß Anspruch 9, wobei der Schritt des in Kontakt Bringens (1), der Wiedergewinnung des Wirtes (2), und der Vermehrungsschritt (3) wiederholt werden.

## Revendications

1. Protéine présentant un peptide, où un peptide est présenté dans une région de présentation constituée des 131^{ème} au 139^{ème} acides aminés dans
(1) la séquence d'acides aminés de la SEQ ID NO : 54 ou
(2) la séquence d'acides aminés de SEQ ID NO : 54, où un à trois acide(s) aminé(s) est/sont substitué(s) dans la séquence du premier au 130^{ème} acides aminés ou la séquence du 140^{ème} au 238^{ème} acides aminés,
dans laquelle le peptide constitué de quatre à quinze acides aminés est inséré dans la séquence du 131^{ème} au 139^{ème} acides aminés, ou substitué à l'une quelconque d'une séquence de 1 à 9 acide(s) aminé(s) dans la séquence du 131^{ème} au 139^{ème} acides aminés ; et une longueur totale d'une séquence d'acides aminés correspondant à la séquence du 131^{ème} au 139^{ème} acides aminés est inférieure ou égale à quinze, et la protéine présente une activité de génération de fluorescence.

2. Protéine de fusion dans laquelle la protéine présentant un peptide selon la revendication 1 et une protéine de surface sont fusionnées.

3. Polynucléotide codant pour la protéine présentant un peptide selon la revendication 1 ou la protéine de fusion selon la revendication 2.

4. Vecteur comprenant le polynucléotide selon la revendication 3.

5. Hôte comprenant le vecteur selon la revendication 4.

6. Banque comprenant l'hôte selon la revendication 5, dans laquelle les peptides présentés représentent un certain nombre de peptides constitués de séquences d'acides aminés aléatoires.

7. Procédé de construction d'une banque, comprenant les étapes qui consistent :
(1) à insérer un polynucléotide codant pour un peptide constitué de 4 à 15 acides aminés dans un vecteur comprenant un polynucléotide codant pour une protéine de présentation d'un peptide ou une protéine de fusion, où la protéine de présentation d'un peptide est fusionnée avec une protéine de surface, pour obtenir un vecteur d'expression, où le peptide est inséré dans la séquence du 131^{ème} au 139^{ème} acides aminés de la région de présentation, ou substitué à l'une quelconque d'une séquence de 1 à 9 acide(s) aminé(s) dans la séquence du 131^{ème} au 139^{ème} acides aminés de la région de présentation ; et une longueur totale de la séquence d'acides aminés correspondant à la séquence du 131^{ème} au 139^{ème} acides aminés est inférieure ou égale à quinze, et
(2) à introduire le vecteur dans un hôte, où la protéine de présentation d'un peptide présente le peptide dans une région de présentation constituée des 131^{ème} au 139^{ème} acides aminés dans la séquence d'acides aminés de la SEQ ID NO. 54, où le peptide constitué de quatre à quinze acides aminés est inséré dans la séquence du 131^{ème} au 139^{ème} acides aminés ou substitué à l'une quelconque d'une séquence de 1 à 9 acide(s) aminé(s) correspondant à la séquence du 131^{ème} au 139^{ème} acides aminés ; et une longueur totale d'une séquence d'acides aminés correspondant à la séquence du 131^{ème} au 139^{ème} acides aminés est inférieure ou égale à quinze.

8. Procédé de criblage d'un peptide se liant à une molécule cible, comprenant les étapes qui consistent :
(1) à mettre en contact la banque de peptides selon la revendication 6 avec la molécule cible,
(2) à récupérer les hôtes se liant à la molécule cible.

9. Procédé de criblage d'un peptide selon la revendication 8, comprenant en outre (3) l'étape qui consiste à faire proliférer les hôtes récupérés.

10. Procédé de criblage d'un peptide selon la revendication 9, dans lequel l'étape de mise en contact (1), l'étape de récupération d'hôtes (2), et l'étape de prolifération (3) sont répétées.


### SEQUENCE LISTING

<110> Tokyo Institute of Technology
<120> Protein presenting peptide and peptide library using the same
<130> TIT-938
<150> JP 2013-090524
   <151> 2013-04-23
<160> 77
<170> PatentIn version 3.1
<210> 1
   <211> 33
   <212> DNA
   <213> Artificial
<220>
   <223> Primer1 for enhanced GFP
<400> 1
   tctagaggat ccatggtgag caagggcgag gag 33
<210> 2
   <211> 60
   <212> DNA
   <213> Artificial
<220>
   <223> Primer2 for enhanced GFP
<400> 2
   tagggcgaat tcttaatggt gatggtgatg atggctgccc ttgtacagct cgtccatgcc 60
<210> 3
   <211> 60
   <212> DNA
   <213> Artificial
<220>
   <223> Primer 3 for mutation
<400> 3
   cacaagttca gcgtgcgcgg cgagggcgag ggcgatgcca ccaacggcaa gctgaccctg 60
<210> 4
   <211> 60
   <212> DNA
   <213> Artificial
<220>
   <223> Primer 4 for mutation
<400> 4
   cagggtcagc ttgccgttgg tggcatcgcc ctcgccctcg ccgcgcacgc tgaacttgtg 60
<210> 5
   <211> 51
   <212> DNA
   <213> Artificial
<220>
   <223> Primer 5 for mutation
<400> 5
   caggagcgca ccatcagctt caaggacgac ggcacctaca agacccgcgc c 51
<210> 6
   <211> 51
   <212> DNA
   <213> Artificial
<220>
   <223> Primer 6 for mutation
<400> 6
   ggcgcgggtc ttgtaggtgc cgtcgtcctt gaagctgatg gtgcgctcct g 51
<210> 7
   <211> 33
   <212> DNA
   <213> Artificial
<220>
   <223> Primer 7 for mutation
<400> 7
   ctgagcaccc agtccgtgct gagcaaagac ccc 33
<210> 8
   <211> 33
   <212> DNA
   <213> Artificial
<220>
   <223> Primer 8 for mutation
<400> 8
   ggggtctttg ctcagcacgg actgggtgct cag 33
<210> 9
   <211> 34
   <212> DNA
   <213> Artificial
<220>
   <223> Primer 9 for mutation
<400> 9
   caagctggag tacaacttca acagccacaa cgtc 34
<210> 10
   <211> 34
   <212> DNA
   <213> Artificial
<220>
   <223> Primer 10 for mutation
<400> 10
   gacgttgtgg ctgttgaagt tgtactccag cttg 34
<210> 11
   <211> 33
   <212> DNA
   <213> Artificial
<220>
   <223> Primer 11 for mutation
<400> 11
   cacaacgtct atatcaccgc cgacaagcag aag 33
<210> 12
   <211> 33
   <212> DNA
   <213> Artificial
<220>
   <223> Primer 12 for mutation
<400> 12
   cttctgcttg tcggcggtga tatagacgtt gtg 33
<210> 13
   <211> 34
   <212> DNA
   <213> Artificial
<220>
   <223> Primer 13 for mutation
<400> 13
   gaagaacggc atcaaggcga acttcaagat ccgc 34
<210> 14
   <211> 34
   <212> DNA
   <213> Artificial
<220>
   <223> Primer 14 for mutation
<400> 14
   gcggatcttg aagttcgcct tgatgccgtt cttc 34
<210> 15
   <211> 34
   <212> DNA
   <213> Artificial
<220>
   <223> Primer 15 for mutation
<400> 15
   caagatccgc cacaacgtgg aggacggcag cgtg 34
<210> 16
   <211> 34
   <212> DNA
   <213> Artificial
<220>
   <223> Primer 16 for mutation
<400> 16
   cacgctgccg tcctccacgt tgtggcggat cttg 34
<210> 17
   <211> 30
   <212> DNA
   <213> Artificial
<220>
   <223> Primer 17 (ml)
<400> 17
   gatgaagttg atgagtacaa cttcaacagc 30
<210> 18
   <211> 18
   <212> DNA
   <213> Artificial
<220>
   <223> Primer 18 (ml)
<400> 18
   caggatgttg ccgtcctc 18
<210> 19
   <211> 48
   <212> DNA
   <213> Artificial
<220>
   <223> Primer 19 (m2)
<400> 19
   aacatcctgg ggcacaagga tgaggttgat ttcaacagcc acaacgtc 48
<210> 20
   <211> 47
   <212> DNA
   <213> Artificial
<220>
   <223> Primer 20 (m2)
<400> 20
   acgttgtggc tgttgaaatc aacctcatcc ttgtgcccca ggatgtt 47
<210> 21
   <211> 30
   <212> DNA
   <213> Artificial
<220>
   <223> Primer 21 (m3)
<400> 21
   gatgaagttg atgacaagca gaagaacggc 30
<210> 22
   <211> 18
   <212> DNA
   <213> Artificial
<220>
   <223> Primer 22 (m3)
<400> 22
   gacgttgtgg ctgttgaa 18
<210> 23
   <211> 36
   <212> DNA
   <213> Artificial
<220>
   <223> Primer 23 (m4)
<400> 23
   ggcggcgatg aagttgatgg gcacaagctg gagtac 36
<210> 24
   <211> 18
   <212> DNA
   <213> Artificial
<220>
   <223> Primer 24 (m4)
<400> 24
   cttgaagtcg atgccctt 18
<210> 25
   <211> 36
   <212> DNA
   <213> Artificial
<220>
   <223> Primer 25 (m5)
<400> 25
   gatgaagttg atggcagcgg gcacaagctg gagtac 36
<210> 26
   <211> 18
   <212> DNA
   <213> Artificial
<220>
   <223> Primer 26 (m5)
<400> 26
   cttgaagtcg atgccctt 18
<210> 27
   <211> 18
   <212> DNA
   <213> Artificial
<220>
   <223> Primer 27 (m6)
<400> 27
   gatgaagttg atggcatc 18
<210> 28
   <211> 18
   <212> DNA
   <213> Artificial
<220>
   <223> Primer 28 (m6)
<400> 28
   gaagtcgatg cccttcag 18
<210> 29
   <211> 36
   <212> DNA
   <213> Artificial
<220>
   <223> Primer 29 (m7)
<400> 29
   ggcgatgaag ttgatggcat cctggggcac aagctg 36
<210> 30
   <211> 18
   <212> DNA
   <213> Artificial
<220>
   <223> Primer 30 (m7)
<400> 30
   gtcgatgccc ttcagctc 18
<210> 31
   <211> 30
   <212> DNA
   <213> Artificial
<220>
   <223> Primer 31 (m12)
<400> 31
   gatgaagttg atgggcacaa gctggagtac 30
<210> 32
   <211> 18
   <212> DNA
   <213> Artificial
<220>
   <223> Primer 32 (m12)
<400> 32
   cttgaagtcg atgccctt 18
<210> 33
   <211> 18
   <212> DNA
   <213> Artificial
<220>
   <223> Primer 33 (m13)
<400> 33
   cacaagctgg agtacaac 18
<210> 34
   <211> 27
   <212> DNA
   <213> Artificial
<220>
   <223> Primer 34 (m13)
<400> 34
   atcaacttca tccttgaagt cgatgcc 27
<210> 35
   <211> 18
   <212> DNA
   <213> Artificial
<220>
   <223> Primer 35 (m14)
<400> 35
   aagctggagt acaacttc 18
<210> 36
   <211> 27
   <212> DNA
   <213> Artificial
<220>
   <223> Primer 36 (m14)
<400> 36
   atcaacttca tccttgaagt cgatgcc 27
<210> 37
   <211> 33
   <212> DNA
   <213> Artificial
<220>
   <223> Primer 37 (m15)
<400> 37
   ggcgatgaag ttgatggcct ggggcacaag ctg 33
<210> 38
   <211> 18
   <212> DNA
   <213> Artificial
<220>
   <223> Primer 38 (m15)
<400> 38
   gaagtcgatg cccttcag 18
<210> 39
   <211> 30
   <212> DNA
   <213> Artificial
<220>
   <223> Primer 39 (m16)
<400> 39
   gatgaagttg atgggcacaa gctggagtac 30
<210> 40
   <211> 18
   <212> DNA
   <213> Artificial
<220>
   <223> Primer 40 (m16)
<400> 40
   gaagtcgatg cccttcag 18
<210> 41
   <211> 33
   <212> DNA
   <213> Artificial
<220>
   <223> Primer 41 (m18)
<400> 41
   tccgacgagg tggacgggca caagctggag tac 33
<210> 42
   <211> 21
   <212> DNA
   <213> Artificial
<220>
   <223> Primer 42 (m18)
<400> 42
   gccgtcctcc ttgaagtcga t 21
<210> 43
   <211> 36
   <212> DNA
   <213> Artificial
<220>
   <223> Primer 43 (m19)
<400> 43
   tccgacgagg tggactccgg gcacaagctg gagtac 36
<210> 44
   <211> 21
   <212> DNA
   <213> Artificial
<220>
   <223> Primer 44 (m19)
<400> 44
   gccgtcctcc ttgaagtcga t 21
<210> 45
   <211> 39
   <212> DNA
   <213> Artificial
<220>
   <223> Primer 45 (m20)
<400> 45
   tccggggacg aggtggactc cgggcacaag ctggagtac 39
<210> 46
   <211> 21
   <212> DNA
   <213> Artificial
<220>
   <223> Primer 46 (m20)
<400> 46
   gccgtcctcc ttgaagtcga t 21
<210> 47
   <211> 42
   <212> DNA
   <213> Artificial
<220>
   <223> Primer 47 (mH1)
<400> 47
   ccggcgagtc cggcggatcg agaagtgggg cacaagctgg ag 42
<210> 48
   <211> 16
   <212> DNA
   <213> Artificial
<220>
   <223> Primer 48 (mH1)
<400> 48
   gccgtcctcc ttgaag 16
<210> 49
   <211> 52
   <212> DNA
   <213> Artificial
<220>
   <223> Primer 49 (mH2)
<400> 49
   cttcaaggag gacggctgcg atggttttta tgcgtgcggg cacaagctgg ag 52
<210> 50
   <211> 52
   <212> DNA
   <213> Artificial
<220>
   <223> Primer 50 (mH2)
<400> 50
   ctccagcttg tgcccgcacg cataaaaacc atcgcagccg tcctccttga ag 52
<210> 51
   <211> 33
   <212> DNA
   <213> Artificial
<220>
   <223> Primer 51 (mH3)
<400> 51
   aaatgctgct atagtctggg gcacaagctg gag 33
<210> 52
   <211> 16
   <212> DNA
   <213> Artificial
<220>
   <223> Primer 52 (mH3)
<400> 52
   gccgtcctcc ttgaag 16
<210> 53
   <211> 717
   <212> DNA
   <213> Artificial
<220>
   <223> sfGFP seq
<400> 53
   atgcgtaaag gcgaggagct gttcaccggg gtggtgccca tcctggtcga gctggacggc      60
   gacgtaaacg gccacaagtt cagcgtgcgc ggcgagggcg agggcgatgc caccaacggc     120
   aagctgaccc tgaagttcat ctgcaccacc ggcaagctgc ccgtgccctg gcccaccctc     180
   gtgaccaccc tgacctacgg cgtgcagtgc ttcagccgct accccgacca catgaagcag     240
   cacgacttct tcaagtccgc catgcccgaa ggctacgtcc aggagcgcac catcagcttc     300
   aaggacgacg gcacctacaa gacccgcgcc gaggtgaagt tcgagggcga caccctggtg     360
   aaccgcatcg agctgaaggg catcgacttc aaggaggacg gcaacatcct ggggcacaag     420
   ctggagtaca acttcaacag ccacaacgtc tatatcaccg ccgacaagca gaagaacggc     480
   atcaaggcga acttcaagat ccgccacaac gtggaggacg gcagcgtgca gctcgccgac     540
   cactaccagc agaacacccc catcggcgac ggccccgtgc tgctgcccga caaccactac     600
   ctgagcaccc agtccgtgct gagcaaagac cccaacgaga agcgcgatca catggtcctg     660
   ctggagttcg tgaccgccgc cgggatcact ctcggcatgg acgagctgta caagtaa        717
<210> 54
   <211> 238
   <212> PRT
   <213> Artificial
<220>
   <223> sfGFP pep
<400> 54
   Met Arg Lys Gly Glu Glu Leu Phe Thr Gly Val Val Pro Ile Leu Val
   1               5                   10                  15
   Glu Leu Asp Gly Asp Val Asn Gly His Lys Phe Ser Val Arg Gly Glu
               20                  25                  30
   Gly Glu Gly Asp Ala Thr Asn Gly Lys Leu Thr Leu Lys Phe Ile Cys
           35                  40                  45
   Thr Thr Gly Lys Leu Pro Val Pro Trp Pro Thr Leu Val Thr Thr Leu
       50                  55                  60
   Thr Tyr Gly Val Gln Cys Phe Ala Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Ser Phe Lys Asp Asp Gly Thr Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Leu Glu Tyr Asn
       130                 135                 140
   Phe Asn Ser His Asn Val Tyr Ile Thr Ala Asp Lys Gln Lys Asn Gly
   145                 150                 155                 160
   Ile Lys Ala Asn Phe Lys Ile Arg His Asn Val Glu Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Val Leu Ser
           195                 200                 205
   Lys Asp Pro Asn Glu Lys Arg Asp His Met Val Leu Leu Glu Phe Val
       210                 215                 220
   Thr Ala Ala Gly Ile Thr His Gly Met Asp Glu Leu Tyr Lys
   225                 230                 235
<210> 55
   <211> 12
   <212> PRT
   <213> Artificial
<220>
   <223> mH1 peptide
<400> 55
   Lys Glu Asp Gly Lys Cys Cys Tyr Ser Leu Gly His
   1               5                   10
<210> 56
   <211> 13
   <212> PRT
   <213> Artificial
<220>
   <223> mH2 peptide
<400> 56
   Lys Glu Asp Gly Cys Asp Gly Phe Tyr Ala Cys Gly His
   1 5 10
<210> 57
   <211> 11
   <212> PRT
   <213> Artificial
<220>
   <223> mH3 peptide
<400> 57
   Lys Glu Asp Gly Phe His Ala His Pro Gly His
   1 5 10
<210> 58
   <211> 45
   <212> DNA
   <213> Artificial
<220>
   <223> Primer 58 (m22)
<400> 58
   gggtccgggg acgaggtgga cgggtccggg cacaagctgg agtac 45
<210> 59
   <211> 21
   <212> DNA
   <213> Artificial
<220>
   <223> Primer 59 (m22)
<400> 59
   gccgtcctcc ttgaagtcga t 21
<210> 60
   <211> 48
   <212> DNA
   <213> Artificial
<220>
   <223> Primer 60 (m8)
<400> 60
   aacatcctgg ggcacgatga ggttgataac ttcaacagcc acaacgtc 48
<210> 61
   <211> 48
   <212> DNA
   <213> Artificial
<220>
   <223> Primer 61 (m8)
<400> 61
   gacgttgtgg ctgttgaagt tatcaacctc atcgtgcccc aggatgtt 48
<210> 62
   <211> 18
   <212> DNA
   <213> Artificial
<220>
   <223> Primer 62 (m9)
<400> 62
   gagtacaact tcaacagc 18
<210> 63
   <211> 30
   <212> DNA
   <213> Artificial
<220>
   <223> Primer 63 (m9)
<400> 63
   atcaacttca tccaggatgt tgccgtcctc 30
<210> 64
   <211> 21
   <212> DNA
   <213> Artificial
<220>
   <223> Primer 64 (m10)
<400> 64
   gttgataagc tggagtacaa c 21
<210> 65
   <211> 21
   <212> DNA
   <213> Artificial
<220>
   <223> Primer 65 (m10)
<400> 65
   ttcatcgttg ccgtcctcct t 21
<210> 66
   <211> 21
   <212> DNA
   <213> Artificial
<220>
   <223> Primer 66 (m11)
<400> 66
   gttgatcaca agctggagta c 21
<210> 67
   <211> 21
   <212> DNA
   <213> Artificial
<220>
   <223> Primer 67 (m11)
<400> 67
   ttcatcgccg tcctccttga a 21
<210> 68
   <211> 27
   <212> DNA
   <213> Artificial
<220>
   <223> Primer 68 (m17)
<400> 68
   aagggcatcg acttcgatga agttgat 27
<210> 69
   <211> 27
   <212> DNA
   <213> Artificial
<220>
   <223> Primer 69 (m17)
<400> 69
   atcaacttca tcgaagtcga tgccctt 27
<210> 70
   <211> 30
   <212> DNA
   <213> Artificial
<220>
   <223> Primer 70 (m21)
<400> 70
   ggggacgagg tggactccgg gcacaagctg 30
<210> 71
   <211> 30
   <212> DNA
   <213> Artificial
<220>
   <223> Primer 71 (m21)
<400> 71
   cagcttgtgc ccggagtcca cctcgtcccc 30
<210> 72
   <211> 36
   <212> DNA
   <213> Artificial
<220>
   <223> Primer 72 (mH4)
<400> 72
   tggtacagct ggctgctggg gcacaagctg gagtac 36
<210> 73
   <211> 18
   <212> DNA
   <213> Artificial
<220>
   <223> Primer 73 (mH4)
<400> 73
   gccgtcctcc ttgaagtc 18
<210> 74
   <211> 36
   <212> DNA
   <213> Artificial
<220>
   <223> Primer 74 (mH5)
<400> 74
   tggtacgcct ggatgctggg gcacaagctg gagtac 36
<210> 75
   <211> 18
   <212> DNA
   <213> Artificial
<220>
   <223> Primer 75 (mH5)
<400> 75
   gccgtcctcc ttgaagtc 18
<210> 76
   <211> 12
   <212> PRT
   <213> Artificial
<220>
   <223> mH4 peptide
<400> 76
   Lys Glu Asp Gly Trp Tyr Ala Trp Met Leu Gly His
   1 5 10
<210> 77
   <211> 12
   <212> PRT
   <213> Artificial
<220>
   <223> mH5 peptide
<400> 77
   Lys Glu Asp Gly Trp Tyr Ser Trp Leu Leu Gly His
   1 5 10